# EUROPEAN PATENT SPECIFICATION

(11) **EP 2 918 265 B1**
(45) Date of publication and mention of the grant of the patent: **11.08.2021**
(21) Application number: 15158619.5
(22) Date of filing: 11.03.2015
(51) Int. Cl.: A61K 9/00, A61K 47/02, A61K 47/36, A61K 47/42

(54) **PRESSURE-SENSITIVE HYDROGEL AND METHOD OF USE**
DRUCKEMPFINDLICHES HYDROGEL UND VERFAHREN ZUR VERWENDUNG
HYDROGEL SENSIBLE À LA PRESSION ET PROCÉDÉ D'UTILISATION

(30) Priority: 14.03.2014 US 201461953153 P
(43) Date of publication of application: 16.09.2015
(73) Proprietor: Taiwan Biomaterial Company Ltd, Hsinchu County 30261 (TW)
(72) Inventor: Liao, Chun Jen, 110 Taipei City (TW); Wang, Wen Hsi, 10082 Taipei City (TW); Wang, Yu Ming, 704 Tainan City (TW)
(74) Representative: Finnegan Europe LLP

(56) References cited:
- WO-A1-2009/105820
- None

## Description

### Field of the Disclosure

Embodiments of the present disclosure include gels, and more particularly, pressure-sensitive gels, such as, but not limited to, pressure-sensitive hydrogels for medical applications.

### Background of the Disclosure

Environmentally sensitive hydrogels have found increasingly broad biomedical applications. Additionally, hydrogels responsive to different stimuli have been used in many clinical applications, such as, e.g., cell therapy and wound dressing.

Gels are nonfluid, semirigid colloidal dispersions of a solid that is expanded in volume via a fluid medium extender distributed throughout. For hydrogels, this fluid medium includes water. Hydrogels may be capable of changing phases, for example, from a fluid to a gel or from a gel to a fluid. These phase changes may be reversible or irreversible, and may be useful, for example, to aid in the transportation (e.g., delivery or removal) of a hydrogel. Different environmental factors may be capable of triggering phase changes in a hydrogel. For example, exposure of a hydrogel to a stimuli may cause the hydrogel to change phases from a fluid to a gel, or vice versa. Such environmental stimuli may include temperature, pH, light, pressure, radiation, and electrical current, for example. The type of stimuli required to transition a hydrogel from one phase to another may affect the applications for which hydrogels are used.

Two major components of biological tissues include water and collagen. Collagen is a structural protein readily found in the bodies of animals, and it is a naturally occurring biopolymer. Collagen is biodegradable, biocompatible, and has a low toxicity profile, making it widely used in medical and clinical applications, including in implantable medical devices, homeostatic gels, tissue augmentation, wound dressings, drug delivery, cardiac valve replacement, blood vessel repair, and surgical sutures. Through various formulation adjustments, morphology rearrangement, physical processing, reconstitution, or chemical cross-linking technology, collagen can be fabricated into sheets, tubes, sponges, powders, or soft fiber fabrics, as well as prepared for use in hydrogels.

Collagen's molecular structure includes three α-chains. Hydrogen bonding and hydrophobic interactions in the structure cause the three α-chains to take a triple-helical conformation. Each triple-helix may associate into a right-handed supercoil, referred to as a collagen microfibril. Each microfibril may be interdigitated with its neighboring microfibrils to a degree that might suggest they are individually unstable, but within collagen fibrils, they may be ordered well enough so as to be crystalline. Because the collagen structure is stabilized by hydrogen bonding and hydrophobic interaction, the structure may be affected by pH value, temperature, and the concentration of electrolytes in the environment. For example, at lower pH values, collagen may be soluble. At room temperature and neutral conditions, collagen may rearrange its microstructure and precipitate as a network fibrillar structure.

Because collagen is more soluble in acidic conditions, collagen may be dissolved in mild acids, such as, e.g., acetic acid, lactic acid, or butyric acid, when preparing collagen solutions or formulations. Dialysis, or other suitable techniques, may then be used to neutralize the acidic aqueous collagen solutions. Further, the rate of collagen fibrillogenesis in a neutral aqueous solution may be affected by temperature. At temperatures above 4° C, collagen may rearrange its microstructure and a gel precipitation may be obtained. Thus, temperature control may play a role in the process of dialysis. If temperature is not regulated, fibrillogenesis may cause inhomogeneity.

Given these characteristics of collagen, temperature-sensitive hydrogels have been prepared by utilizing the ability of temperature to induce structural transitions. Collagen hydrogel materials have been used in clinical applications, including as derma filler, in drug delivery, and for cell therapy. Although temperature-sensitive collagen hydrogels have been used in clinical applications, their manufacture may be time-consuming and energy-consuming, and the hydrogels may be unstable in clinical applications.

Currently available pressure-sensitive hydrogels are based on similar mechanisms as temperature-sensitive hydrogels. Without being limited to a particular theory, when some hydrogel polymers are placed in hydrostatic conditions, pressure changes may cause a variation in lower critical solution temperature ("LCST"), as well as a change in the volume of a hydrogel. Such may be the case with hydrogel polymers like poly(N-iso-propylacrylamide), poly(N-n-propylacrylamide), poly(N,N-diethylacrylamide), and poly(N-isopropylacrylamide), for example. When the temperature is close to LCST, these hydrogels may be stacked and compressed under low hydrostatic pressure and then may swell under high hydrostatic pressure. However, this type of pressure-sensitive LCST mechanism may only work with certain polymers, such as those mentioned above, and may not work with other polymers, such as collagen.

Accordingly, there exists a need for a compact, cost-effective, reliable, and easy-to-control hydrogel capable of reacting structurally to changes in pressure. In addition, there exists a need for a new type of pressure-sensitive hydrogels capable of utilizing a broader range of polymers to expand the potential medical applications of pressure-sensitive hydrogels. Embodiments of the present disclosure described herein aim to overcome one or more of these, and other, limitations in the prior art in an economical and safe fashion.

WO 2009/105820 A1 relates to elastin-based hydrogels that are formed under pressurized conditions, and discloses a method for producing a hydrogel including: providing a vessel including a liquid phase, the liquid phase including elastin material; pressurizing the vessel with a gas to dissolve the gas into the liquid phase; and depressurizing the vessel to release gas from the liquid phase, thereby producing the hydrogel. However, the formation of the hydrogel is caused by cross linking the coacervated elastin.

### II. Summary of the Disclosure

Embodiments of the present disclosure provide a pressurized hydrogel composition according to claim 1, and a composition according to claim 5.

In accordance with one embodiment, a composition may include a liquid solvent, a polymer, and an acid gas. The composition may be capable of having a fluid phase in which the acid gas is dissolved in the solvent and the polymer is dissolved in the solvent, and the composition may be capable of having a gel phase in which the acid gas is not dissolved in the liquid solvent and the polymer is precipitated out of the solvent.

In accordance with another embodiment, a composition may include a hydrogel, wherein the hydrogel exists as a fluid having a first pH at a first pressure and exists as a gel having a second pH at a second pressure.

Various embodiments of the composition may include one or more of the following features: the hydrogel may include a solvent, a polymer, and an acid gas; at the first pressure the polymer and the acid gas may be dissolved in the solvent, and at the second pressure the polymer and the acid gas may be substantially precipitated out of the solvent; the first pH may be lower than the second pH; and the first pressure may be higher than the second pressure.

In accordance with yet another embodiment of the disclosure, a method of making a pressure-sensitive hydrogel composition may include exposing a hydrogel formed of a polymer and a solvent to an acid gas and subjecting the hydrogel and the acid gas to an increase in pressure to dissolve the acid gas in the solvent to form a more acidic solution. The more acidic solution may cause the polymer to dissolve into the solution to form a fluid, and subjecting the hydrogel to a decrease in pressure allows the acid gas to come out of solution to form a less acidic solution, wherein the less acidic solution causes the polymer to precipitate out of the solvent to form a gel.

In accordance with another embodiment of the present disclosure, a method of repairing tissue may include delivering a pressurized hydrogel composition to a target region of the tissue and allowing the hydrogel to de-pressurize. The hydrogel may be formed of a mixture of a polymer, a solvent, and an acid gas. The pressurized hydrogel may be in a liquid state, and the depressurized hydrogel may be in a gel state.

It is to be understood that the present disclosure is not limited in its application to the details of construction and to the arrangements of the components set forth in the following description or illustrated in the drawings. The present disclosure is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be used as a basis for designing other structures, methods, and systems for carrying out the several purposes of the present disclosure.

### III. Brief Description of the Drawings

The accompanying drawings, which are incorporated in and constitute a part of this specification, illustrate exemplary embodiments of the present disclosure, and together with the description, serve to explain the principles of the disclosure.
FIG. 1 depicts the appearance of six exemplary hydrogels in a gel state, according to embodiments of the present disclosure;
FIG. 2 depicts the exemplary hydrogels of FIG. 1 approximately 30 minutes after being released from a syringe;
FIG. 3 depicts the appearance of five exemplary hydrogels in a gel state, according to embodiments of the present disclosure;
FIG. 4 depicts the exemplary hydrogels of FIG. 3 approximately 30 minutes after being released from a syringe;
FIG. 5 depicts the appearance of seven exemplary hydrogels in a gel state, according to embodiments of the present disclosure;
FIG. 6 depicts the exemplary hydrogels of FIG. 5 approximately 30 minutes after being released from a syringe;
FIG. 7 depicts the appearance of four exemplary hydrogels in a gel state, according to embodiments of the present disclosure;
FIG. 8 depicts the exemplary hydrogels of FIG. 7 approximately 30 minutes after being released from a syringe;
FIG. 9 depicts the appearance of four exemplary hydrogels in a gel state, according to embodiments of the present disclosure;
FIG. 10 depicts the exemplary hydrogels of FIG. 9 approximately 30 minutes after being released from a syringe;
FIG. 11a depicts the appearance of an exemplary hydrogel in a fluid state, according to embodiments of the present disclosure;
FIG. 11b depicts the appearance of the exemplary hydrogel of FIG. 11a in a gel state;
FIG. 12a depicts the appearance of the exemplary hydrogel of FIG. 11a in a fluid state;
FIG. 12b depicts the appearance of the exemplary hydrogel of FIG. 11a in a gel state; and
FIG. 13 depicts an exemplary drug delivery hydrogel, according to an exemplary embodiment of the present disclosure.

### IV. Detailed Description

Reference will now be made in detail to the exemplary embodiments of the present disclosure described below and illustrated in the accompanying drawings. Wherever possible, the same reference numbers will be used throughout the drawings to refer to same or like parts.

While the present disclosure is described herein with reference to illustrative embodiments of pressure-sensitive hydrogels for medical applications, it is understood that the devices and methods of the present disclosure may be employed for any suitable application, including, but not limited to, agricultural, industrial, environmental, or commercial applications. Further, while collagen hydrogels using polymer networks are described, any suitable network component, including colloidal networks, e.g., may be utilized, and the hydrogels may include any suitable natural or synthetic polymers. Likewise, gels according to the present disclosure are not limited to *hydro*gels, and may include any other suitable liquid other than, or in addition to, water. Those having ordinary skill in the art and access to the teachings provided herein will recognize additional modifications, applications, embodiments, and substitution of equivalents that all fall within the scope of the disclosure. Accordingly, the disclosure is not to be considered as limited by the foregoing or following descriptions.

As used herein, the term "fluid" may refer to a substance whose molecules move freely past one another and has no fixed shape, e.g., a gas or a liquid. The term "dissolve" may refer to either partial or complete dissolving of a solute into a solvent, and "solution" may refer to a mixture created when a solute dissolves (either partially or fully) into a solvent. As used herein, the term "hydrogel" will be understood to mean both a gel in which water is a solvent, as well as a gel in which one or more solvents other than water are used.

The solubility of an acid gas may increase as the pressure of the surrounding environment increases. When not dissolved into solution, acid gases may not substantially affect the pH of surrounding liquids. When dissolved within a liquid, acid gases may increase the acidity of the resulting solution, thus, decreasing the solution's pH value. Accordingly, under higher pressures, an acid gas exposed to a liquid may become more soluble, and as a result, may dissolve into solution. In turn, the dissolving of the acid gas into solution may decrease the pH of the solution. If the pressure is then decreased, the acid gas may come back out of solution, reducing the solution's acidity and thus raising the solution's pH value.

Embodiments of the present disclosure utilize the solubility traits of synthetic and/or natural polymers to create new pressure-sensitive hydrogels. In some embodiments, hydrogels may include substantially neutral, aqueous polymer solutions. The polymers may be soluble in acidic conditions and may be insoluble or less soluble as the pH becomes less acidic. The aqueous polymer solutions may be exposed to acid gas. When exposed to higher pressures, the acid gas may be forced to dissolve into the aqueous polymer solution. As the acid gas dissolves into the solution, it may lower the pH value of the resulting solution, which may in turn cause the polymer to substantially dissolve into the acidic, aqueous solution. This may create a fluid solution of dissolved acid gas and polymer. A subsequent decrease in pressure may decrease the solubility of the acid gas, causing the acid gas to come out of solution. The precipitation of acid gas out of solution may in turn increase the pH value of the aqueous solution, e.g., in some examples, restoring it to a substantially neutral pH, or to a less-acidic or basic pH. The decrease in the solution's acidity may in turn cause the polymer to precipitate out of solution, causing the mixture to transition from a fluid to a gel. In this way, synthetic or natural polymers may be used to form hydrogels capable of transitioning from a fluid phase to a gel phase in response to a pressure stimulus using novel mechanisms different than those previously utilized.

Embodiments of the present disclosure may take advantage of the different solubility traits of different synthetic polymers and/or natural polymers, such as collagen, to create pressure-sensitive hydrogels. As discussed previously, collagen may be soluble under acidic conditions with pH values of less than neutral. In some embodiments, a collagen hydrogel may include collagen and a solvent (e.g., water) exposed to an acid gas under ambient pressure. Under ambient pressure, the acid gas may not dissolve, or may only partially dissolve, into solution, and thus exposure to the acid gas under ambient pressure may not significantly decrease the pH value of the solution. Due to the higher (e.g., substantially neutral) pH, the collagen may not dissolve into solution at ambient pressure, or may only partially dissolve, or may form an inhomogeneous state. At this point, the collagen hydrogel may exist in a gel phase.

Exposing the mixture to an increase in pressure may increase the solubility of the acid gas, creating an acid-gas solution and causing the pH of the resulting solution to drop. The decrease in pH may cause the collagen to dissolve, e.g., into a substantially homogenous solution. Causing the collagen to dissolve into solution may transition the hydrogel to a fluid state. Thus, under relatively lower-pressure conditions, the hydrogel may transition to a gel phase, and under relatively higher-pressure conditions, the hydrogel may transition to a fluid phase.

A subsequent decrease in pressure may once again decrease the solubility of the acid gas, causing it to come out of solution, and causing the pH value of the solution to rise. In some embodiments, the pH value of the solution may be neutralized, or may become less acidic or basic, as the acid gas comes out of solution. This may in turn cause the collagen to precipitate, at least in part, and may cause the mixture to transition back from a fluid to a gel. Accordingly, a pressure-sensitive hydrogel may be prepared according to the mechanisms described above.

As described above, the pressure-sensitive hydrogels exist in a liquid state when acid gas is forced into solution under increased pressures, causing the pH value of the solution to drop. The hydrogels transition to a gel state when the acid gas comes out of solution, and the polymer precipitates out due to the increase in pH value. Because the hydrogel sets to form a gel as the acid gas comes out of solution, gas may be trapped as it comes out of solution, forming bubbles within the set hydrogel. The formation of bubbles may cause the hydrogel to be porous when in the gel phase. In some embodiments, the bubbles may not connect with one another, and while the hydrogel may be porous, it may simultaneously be able to act as a sealant. In some embodiments, the porous nature of the gel may make the resulting hydrogel breathable, i.e., allowing air and/or gases to pass through, while in some embodiments the resulting hydrogel may be impermeable to air and/or gases

Embodiments of the present disclosure may include any suitable natural or synthetic polymer or polymers that are more soluble under a first range of pH values, e.g., more acidic conditions having a relatively lower pH, and are less soluble under a second, different range of pH values, e.g., less acidic conditions, neutral conditions, or basic conditions having a relatively higher pH compared to the first pH range. Under the first range of pH values, a polymer hydrogel may exist in a fluid state, and under the second range, the polymer hydrogel may transition to a gel state. A hydrogel made from such a polymer may include or may be exposed to an acid gas, and as the solubility of the acid gas increases with an increase in pressure, the increased acidity of the resulting acid-gas solution may increase the solubility of the polymer, causing the hydrogel to transition to a fluid phase. The pH of the solution may thus be controlled via pressure, and accordingly, pressure may be manipulated to trigger transitioning of the hydrogel from a gel state to a fluid state, and/or vice versa. Accordingly, pressure-sensitive hydrogels may be created by manipulating the ability to dissolve or precipitate certain polymers based on changes in pH and the ability to manipulate pH by forcing acid gases into and out of solution in response to changes in pressure.

As discussed above, any suitable polymers or combination of polymers may be used to form a pressure-sensitive hydrogel. For example, polymers such as gelatin, cellulose, hyaluronic acid, chitosan, casein, alginate, fibrinogen, or thrombin may be used. The type or types of polymers used to form a pressure-sensitive hydrogel may be determined, at least in part, on the application of the hydrogel, the type of acid gas used, or the type of liquid solvent used. In turn, the type of polymer selected may affect the pH ranges needed to dissolve or precipitate the polymer into and out of solution. For example, an acidic pH of less than 6 may be used to dissolve the polymer, and an approximately neutral pH level of 6 or greater may be used to precipitate the polymer out of solution. In some embodiments, a more acidic pH and a less-acidic pH may be used to dissolve and precipitate the polymer, respectively, or an acidic pH and a basic pH may be used to dissolve and precipitate the polymer, respectively. In some embodiments, the difference between the dissolving pH and the precipitating pH may be small, while in other embodiments, the difference between the dissolving pH and the precipitating pH may be larger. The type of polymer used and the ranges of pH values required to dissolve or precipitate that polymer may affect the liquid solvent or the acid gas used in conjunction with that polymer to form a suitable pressure-sensitive hydrogel.

In some embodiments, multiple polymers may be used to form a single hydrogel. For example, in some embodiments, polymers with different solubility characteristics at different pH ranges may be included in the same hydrogel. Thus, exposure to different pH ranges may cause the hydrogel to transition to a gel state at different pressures as different amounts of acid gas are forced to dissolve into solution because of the change in pressure. In some embodiments, the polymers used may form gels of different firmness. For example, exposing a multi-polymer hydrogel to a first set of pressures may cause a first amount of acid gas to dissolve, creating a first pH range that causes a first polymer, but not a second polymer, to dissolve into solution. The second, non-dissolved polymer may cause a first gel state with a first viscosity. Exposing the hydrogel to a second set of pressures may cause a second, lesser amount of acid gas to dissolve, creating a second pH range and causing the first polymer to also precipitate out of solution, in addition to, or instead of, the second polymer. The first polymer may precipitate to form a second gel state having a second, different viscosity from the first gel state. Thus, a pressure-sensitive hydrogel may be created that is capable of transitioning to gel states with different viscosities, depending on the amount of pressure that the hydrogel is subjected to.

In some embodiments, hydrogels incorporating more than one polymer may provide redundancy. For example, some polymers may denature under different conditions, for example, different temperatures or pH ranges, or may have different shelf-lives. The inclusion of multiple types of polymers in a single hydrogel may increase the overall stability of the hydrogel or increase the likelihood that the hydrogel will transition from a fluid to a gel state as intended when subjected to a change in pressure. Such hydrogels may be useful, for example, when delivering the hydrogel to different environments (e.g., different areas in the body, when used in conjunction with different treatments or drugs), or when distribution or storage conditions to an end user or by a user of the hydrogel may be unpredictable or uncontrollable.

Further, any suitable type or types of acid gas may be used with embodiments of the present disclosure. For example, hydrogen chloride, chlorine, sulfur dioxide, thiosulfate, nitrogen dioxide, hydrogen sulfide, hydrogen fluoride, carbon dioxide, or any suitable combination thereof, may be used as a suitable acid gas. In some embodiments, weaker gases may be utilized because they may be easier to force into and precipitate out of solution. Further, in some embodiments, the acid gas may be in a solid or a liquid state when introduced to the mixture. For example, if carbon dioxide is used as the acid gas, it may be introduced to the fluid hydrogel mixture either in a gaseous, a liquid state, or solid state (i.e., dry ice). If introduced in a liquid or solid state, carbon dioxide may either completely or partially (so that some solid or liquid still remains) transition to an acid gas and then dissolve into solution. The pH values achieved by forcing different acid gases into solution may vary, for example, the pH values may range from approximately 7 to approximately 2, depending on the type of acid gas used. Thus, the type of acid gas used may affect the type of polymer used. For example, collagen may dissolve at a pH of approximately 3 and may precipitate at a pH of approximately 5. The type or types of acid gas used may be determined, at least in part, by the hydrogel application, the type of solvent used, or the type of polymer used.

In some embodiments, the type of acid gas used may affect the amount of pressure required to dissolve the acid gas into and out of solution, and thus the pressure at which the hydrogel will transition to a gel state or a liquid state. In some embodiments, the type of acid gas used may affect the pH change induced when the acid gas is dissolved. In some embodiments, multiple acid gases may be used in a single hydrogel. For example, a first acid gas may dissolve into solution under a first range of pressures and/or may create a first pH range when dissolved. A second acid gas may dissolve into solution under a second range of pressures and/or may cause a second pH range when dissolved. Accordingly, the different acid gases may cause the hydrogel to transition to fluid or a gel at different pressure ranges. The different acid gases may also cause more or less dissolving of the polymer, or more or less homogeneity of the polymer solution. This may in turn affect the viscosity of the hydrogel when transitioning to the gel state. In some embodiments, using multiple acid gases may also facilitate the use of multiple polymers within a hydrogel.

In addition, any suitable type of liquid solvent may be used to form a hydrogel according to the present disclosure. While the present disclosure refers primarily to hydrogels, and thus brings to mind the use of water as the solvent, any suitable liquid solvent may be used, for example, saline solutions, buffer solutions, alcohol (e.g., methanol or ethanol), esters, ketones, hydrocarbons, and ether, or any suitable combination thereof. In some embodiments, a buffer solution (e.g., Hank's Balanced Salt Solution, Dulbecco's Phosphate-Buffered Saline, Ringer Solution, Tri-HCI, etc.) may be used alone or in combination with another solvent, e.g., water. The type or types of solvents used may be determined, at least in part, by the hydrogel application, the type of acid gas used, or the type of polymer used. In turn, the type of acid gas used, the type of polymer used, and the ranges of pH needed to dissolve or precipitate the polymer may affect the type of solvent chosen.

The amount of pressure necessary to transition a hydrogel from a fluid state to a gel state and/or back to a fluid state may depend, at least in part, on the type of solvent, polymer, or acid gas used in a given hydrogel embodiment. In some embodiments, the transition point from a fluid to a gel may be defined as a predetermined amount above ambient pressure, and the hydrogel may exist as a gel at a pressure substantially equal to ambient pressure. In other embodiments, the transition point may be above ambient pressure, while the hydrogel may also exist as a gel above ambient pressure, or the transition point may be below ambient pressure, or the transition point may be at substantially ambient pressure and the hydrogel may exist as a gel below ambient pressure. Further, as will be recognized, "ambient pressure" may be relative depending on geographic location, and the exact transition point may vary slightly depending on ambient conditions, for example, geographic location or temperature.

The amount of pressure necessary to transition between the fluid and gel states may be chosen, at least in part, according to the intended application of the hydrogel. In some embodiments, the transition from a fluid to a gel or from a gel to a fluid may be gradual and may occur over a range of transition pressures. In other embodiments, the hydrogel phase transition may occur more abruptly as a given pressure threshold is crossed. In some embodiments, the transition point from a fluid to a gel phase may be different than the transition point of a gel to a fluid phase or vice versa, for example, in abruptness or in the exact pressure required. The gradual or abrupt nature of the transition may be affected, at least in part, by the polymer or solvent used, or by environmental conditions, such as the surrounding temperature or humidity, for example. In some embodiments, the transition from a fluid to a gel may take less than a minute, while in some embodiments, the transition may take several minutes. For example, the transition may take approximately 30 seconds, 1 to 2 minutes, 10 minutes, or up to two hours or longer.

Additionally, according to the gas laws, there is a known relationship between temperature and pressure. Thus, both of these variables may affect the gelling properties of a pressure-sensitive hydrogel. Under different temperatures and different pressures, hydrogels may have different gelling rates or different gelling properties (e.g., viscosity, handling, etc.). For example, exposing a hydrogel to a higher temperature may cause the hydrogel to transition to a gel more quickly than the same hydrogel when exposed to a lower temperature. The effects of temperature and pressure may also depend at least in part on the type of polymer included in a given hydrogel. For example, a collagen-based hydrogel may take approximately 1 to 2 minutes to gel at approximately body temperature (roughly 37.5°C) and a pressure of 1 atm. By changing the temperature, different gels may have different optimal transition pressures. Accordingly, by controlling the temperature in addition to the pressure, the gelling rate and/or the gelling characteristics may be more finely controlled. The exact optimum pressure ranges for transitioning a hydrogel and for maintaining either a gel or liquid state may depend on the polymer used and the acid gas used and may be unpredictable.

The transition of an exemplary, pressure-sensitive hydrogel from a fluid to a gel may be reversible in some embodiments. For example, exposing the hydrogel to a decrease in pressure may cause the hydrogel to transition from a fluid to a gel, and subsequently increasing the pressure may cause the hydrogel to transition from a gel back to a fluid. In some embodiments, the transition may be irreversible. For example, exposing the hydrogel to a decrease in pressure may cause the hydrogel to transition from a fluid to a gel, and subsequently increasing the pressure may not cause the hydrogel to transition back to a fluid. In such embodiments, once transitioned, the hydrogel may maintain its gel state independent of the surrounding pressure.

### Examples of Pressure-Sensitive Hydrogels

### Examples 1-6

A series of six hydrogels was formed by mixing 0.2 g of collagen and 4 g of solid carbon dioxide (dry ice) with one of the following buffer solutions in a 20 ml stainless-steel bottle: (i) 10 ml of Hank's Balanced Salt Solution (HBSS), (ii) Dulbecco's Phosphate-Buffered Saline (DPBS), (iii) normal saline, (iv) Ringer Solution, (v) Tri-HCI buffer, and (vi) water. The bottle was sealed with a screwed valve in order to prevent leakage of the carbon dioxide. At equilibrium, under room temperature (approximately 30°C), the dry ice inside of the bottle was transformed into either a homogeneous gas state or a gas-liquid coexistent state. The bottle was allowed to remain at room temperature for approximately 18-20 hours.

The valve was then unscrewed, and the collagen was released from the stainless-steel bottle, allowing the hydrogel to return to atmospheric pressure. The gelling ability and handling properties of each of the released gels was analyzed, and the visual appearance of each gel was observed and photographed.

### Gelling Ability

Gelling ability, or gelation property, is generally defined as the ability of a hydrogel to form a gel and retain its shape over a period of time. Gelling ability may be assessed by visual observation of the shape and ability of the gel to maintain a given shape once set. This visual observation can then be scored and/or ranked. Gelling ability was assessed according to the following procedure. For each of the six hydrogels, 0.39 ml of the gel was put into a 96-well plate. Triplicates were made for all groups. The plate was put into an ELISA reader after approximately 10 minutes of incubation at room temperature, and results were read with a measurement wavelength of 450 nm.

Table 1 (below) shows the semi-qualitative evaluation of gelling ability. Gelation property measures the ability of a gel to maintain a given shape after gelling. For example, a '0' rating may indicate that while a hydrogel gelled, it failed to keep its shape.

**Table 1. Semi-Qualitative Evaluation of Gelling Ability**

| **OD 450 nm** | **Gelation Property** |
|---|---|
| 4.00 or more | +++ |
| 3.00-3.99 | ++ |
| 2.50-2.99 | + |
| 2.49 or less | 0 |

### Handling Property

Handling property is generally defined as the ability of a hydrogel to gel at the desired time. For example, if a delivery device is used to store and then release a hydrogel, a hydrogel that gels while still in the delivery device would not have good handling properties, because it would have set - and therefore its shape would have been determined - in the device rather than when released from the device for use in a given application. Handling property is a measure of the manipulability and operation of a given hydrogel. Handling property was assessed according to the following procedure. For each of the six hydrogels, 1.5 ml of the gel was put into a syringe of which the tip had been removed. The gel-filled syringe was incubated at room temperature for approximately 10 minutes. The gel was pushed out from the syringe, and the integrity of the gel structure was evaluated and observed.

If the gel could remain intact as a cylinder shape after being pushed out of the syringe, this indicated that the gel was not set until it was released from the stainless-steel bottle. If the gel broke into small pieces after being pushed out of the syringe, this indicated that the gel set before it was released from the bottle and could not be modified into a desired shape. Photographs were taken after pushing the gels out of the syringe for observation of visual appearance. Photographs were again taken of the gels after approximately 30 minutes of incubation at room temperature.

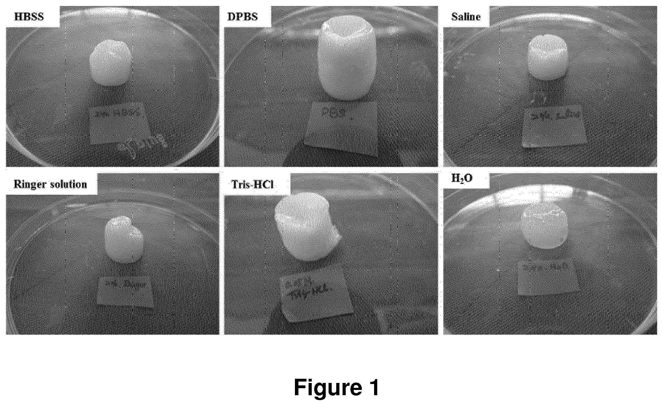

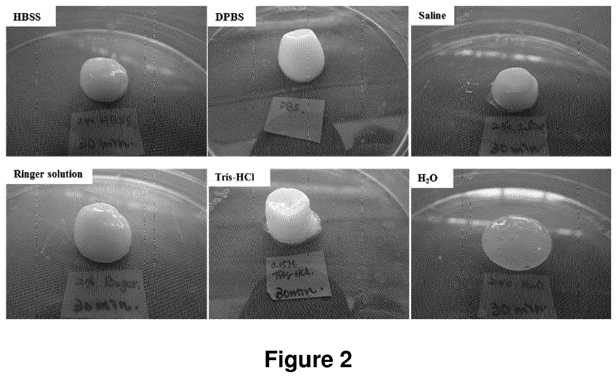

Table 2 (below) shows the results of gelling ability, handling property, and visual appearance of the gels released from the stainless-steel bottle containing solid carbon dioxide with the various buffer solutions. The appearances of the resulting gels are depicted in Figures 1 and 2. Figure 1 shows the appearance of collagen mixed with solid carbon dioxide and each of the six buffer solutions after approximately 10 minutes of gelling in the syringe. Figure 2 depicts the appearance of collagen mixed with solid carbon dioxide and each of the six buffer solutions approximately 30 minutes after being pushed out of the syringe.

**Table 2. Gelling Ability and Handling of Buffer Solution Gels**

| | Example | 1 | 2 | 3 | 4 | 5 | 6 |
|---|---|---|---|---|---|---|---|
| Collagen(g) | | 0.2 | 0.2 | 0.2 | 0.2 | 0.2 | 0.2 |
| CO2 (g) | | 4 | 4 | 4 | 4 | 4 | 4 |
| HBSS(ml) | | 10 | | | | | |
| | Potassium Chloride (5.33mM) | | | | | | |
| | Potassium Phosphate monobasic (0.441mM) | | | | | | |
| | Sodium Bicarbonate (4.17mM) | | | | | | |
| | Sodium Chloride (137.93mM) | | | | | | |
| | Sodium Phosphate dibasic anhydrous(0.338mM) | | | | | | |
| | D-Glucose (5.56mM) | | | | | | |
| DPBS(ml) | | | 10 | | | | |
| | Potassium Chloride (2.67mM) | | | | | | |
| | Potassium Phosphate monobasic (1.47mM) | | | | | | |
| | Sodium Chloride (137.93mM) | | | | | | |
| | Sodium Phosphate dibasic (8.06mM) | | | | | | |
| Saline(ml) | | | | 10 | | | |
| | Sodium Chloride (153.84mM) | | | | | | |
| Ringer solution(ml) | | | | | 10 | | |
| | Sodium Chloride (147mM) | | | | | | |
| | Potassium Chloride (4.02mM) | | | | | | |
| | Calcium Chloride Dihydrate (2.24mM) | | | | | | |
| Tris-HCl buffer(ml) | | | | | | 10 | |
| | Tris base(50mM) | | | | | | |
| Purified water(ml) | | | | | | | 10 |
| Operation | | Yes | Yes | Yes | Yes | Yes | Yes |
| OD450nm | | 2.762 | 3.851 | 3.088 | 3.096 | 2.706 | 2.038 |
| Gelation property | | + | ++ | ++ | ++ | + | 0 |

### Examples 7-11

A series of five hydrogels was formed by mixing 10 ml of HBSS and 4 g of solid carbon dioxide (dry ice) with one of the following amounts of collagen in a 20 ml stainless-steel bottle: (i) 0.05 g of collagen, (ii) 0.1 g of collagen, (iii) 0.2 g of collagen, (iv) 0.3 g of collagen, and (v) 0.4 g of collagen. The bottle was sealed with a screwed valve in order to prevent leakage of the carbon dioxide. At equilibrium, under room temperature (approximately 30°C), the dry ice inside of the bottle was transformed into either a homogeneous gas state or a gas-liquid coexistent state. The bottle was allowed to remain at room temperature for approximately 18-20 hours.

The valve was then unscrewed, and the collagen was released from the stainless-steel bottle, allowing the hydrogel to return to atmospheric pressure. The gelling ability and handling properties of each of the released gels was analyzed, and the visual appearance of each gel was observed and photographed.

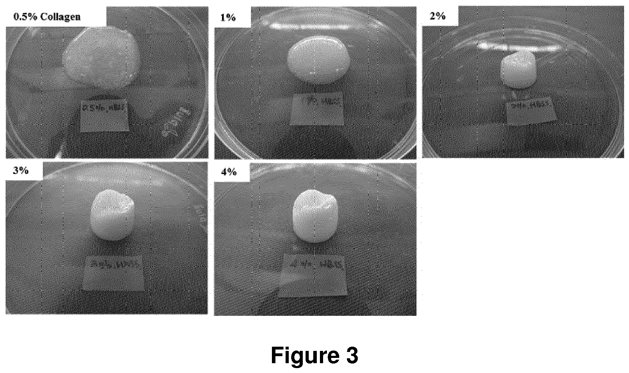

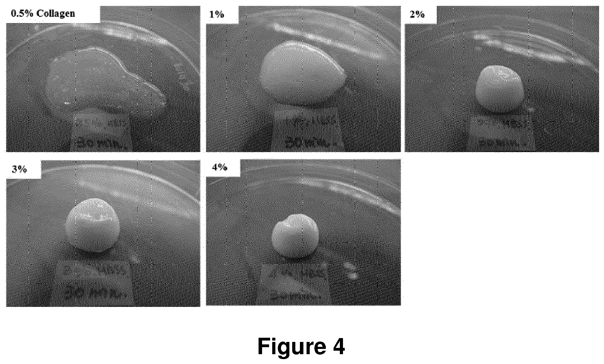

Table 3 (below) shows the results of gelling ability, handling property, and visual appearance of the gels released from the stainless-steel bottle containing solid carbon dioxide and HBSS with the different amounts of collagen. The appearances of the resulting gels are depicted in Figures 3 and 4. Figure 3 shows the appearance of solid carbon dioxide and HBSS mixed with the different amounts of collagen after approximately 10 minutes of gelling in the syringe. Figure 4 depicts the appearance of solid carbon dioxide and HBSS with the different amounts of collagen approximately 30 minutes after being pushed out of the syringe.

**Table 3. Gelling Ability and Handling of Collagen Gels**

| | Example | 7 | 8 | 9 | 10 | 11 |
|---|---|---|---|---|---|---|
| Collagen(g) | | 0.05 | 0.1 | 0.2 | 0.3 | 0.4 |
| CO2 (g) | | 4 | 4 | 4 | 4 | 4 |
| HBSS(ml) | | 10 | 10 | 10 | 10 | 10 |
| | Potassium Chloride (5.33mM) | | | | | |
| | Potassium Phosphate monobasic (0.441mM) | | | | | |
| | Sodium Bicarbonate (4.17mM) | | | | | |
| | Sodium Chloride (137.93mM) | | | | | |
| | Sodium Phosphate dibasic | | | | | |
| | anhydrous(0.338mM) | | | | | |
| | D-Glucose (5.56mM) | | | | | |
| Operation | | Yes | Yes | Yes | Yes | Yes |
| OD450nm | | 1.602 | 2.989 | 2.995 | 3.697 | 4 |
| Gelation property | | 0 | + | + | ++ | +++ |

### Examples 12-18

A series of seven hydrogels was formed by mixing 10 ml of HBSS and 2 g of collagen with one of the following amounts of solid carbon dioxide (dry ice) in a 20 ml stainless-steel bottle: (i) 0.5 g of solid carbon dioxide, (ii) 1 g of solid carbon dioxide, (iii) 2 g of solid carbon dioxide, (iv) 3 g of solid carbon dioxide, (v) 4 g of solid carbon dioxide, (vi) 5 g of solid carbon dioxide, and (vii) 6 g of solid carbon dioxide. The bottle was sealed with a screwed valve in order to prevent leakage of the carbon dioxide. At equilibrium, under room temperature (approximately 30°C), the dry ice inside of the bottle was transformed into either a homogeneous gas state or a gas-liquid coexistent state. The bottle was allowed to remain at room temperature for approximately 18-20 hours.

The valve was then unscrewed, and the collagen was released from the stainless-steel bottle, allowing the hydrogel to return to atmospheric pressure. The gelling ability and handling properties of each of the released gels was analyzed, and the visual appearance of each gel was observed and photographed.

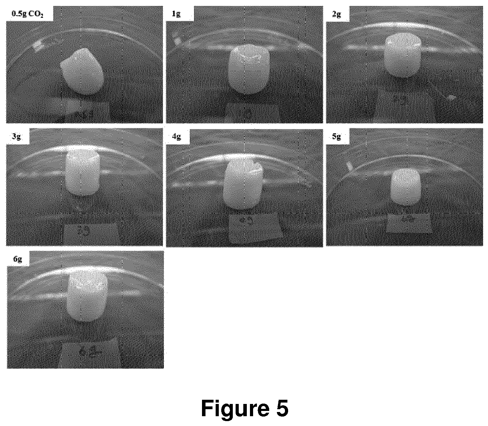

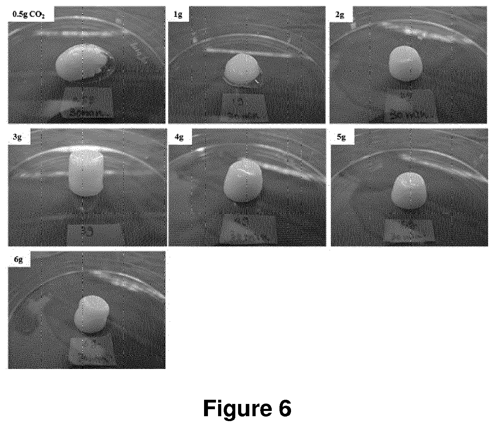

Table 4 (below) shows the results of gelling ability, handling property, and visual appearance of the gels released from the stainless-steel bottle containing collagen and HBSS with the different amounts of solid carbon dioxide. The appearances of the resulting gels are depicted in Figures 5 and 6. Figure 5 shows the appearance of collagen and HBSS mixed with the different amounts of solid carbon dioxide after approximately 10 minutes of gelling in the syringe. Figure 6 depicts the appearance of collagen and HBSS with the different amounts of solid carbon dioxide approximately 30 minutes after being pushed out of the syringe.

Using different amounts of carbon dioxide to evaluate pH-sensitivity may create different pressures if the remaining variables, e.g., bottle size, are kept the same across experiment groups. Accordingly, Table 4 also includes the theoretical pressure calculation for each experiment group. For example, while not measured during the experimental trials, and while not bound to a particular range, the pressure may be approximately 55.5 atm when 1 g of carbon dioxide is mixed with 10 ml of HBSS, and the carbon dioxide may begin to dissolve into solution at approximately 56 atm and 20°C.

**Table 4. Gelling Ability and Handling of CO₂ Gels**

| | Example | 12 | 13 | 14 | 15 | 16 | 17 | 18 |
|---|---|---|---|---|---|---|---|---|
| Collagen(g) | | 0.2 | 0.2 | 0.2 | 0.2 | 0.2 | 0.2 | 0.2 |
| CO2 (g) | | 0.5 | 1 | 2 | 3 | 4 | 5 | 6 |
| Pressure@20°C(atm) | | 27.77 | 55.5 | 56 | 56 | 56 | 56 | 56 |
| HBSS(ml) | | 10 | 10 | 10 | 10 | 10 | 10 | 10 |
| | Potassium Chloride (5.33mM) | | | | | | | |
| | Potassium Phosphate monobasic (0.441mM) | | | | | | | |
| | Sodium Bicarbonate (4.17mM) | | | | | | | |
| | Sodium Chloride (137.93mM) | | | | | | | |
| | Sodium Phosphate dibasic anhydrous(0.338mM) | | | | | | | |
| | D-Glucose (5.56mM) | | | | | | | |
| Operation | | Yes | Yes | Yes | Yes | Yes | Yes | Yes |
| OD450nm | | 2.836 | 2.831 | 2.888 | 2.865 | 2.882 | 2.879 | 2.946 |
| Gelation property | | + | + | + | + | + | + | + |

### Examples 19-22

A series of four hydrogels was formed by mixing 10 ml of water, 0.2 g of collagen, and 4 g of solid carbon dioxide with one of the following amounts of sodium bicarbonate (NaHCO₃) in a 20 ml stainless-steel bottle: (i) 3 mg of NaHCO₃, (ii) 7.5 mg of NaHCO₃, (iii) 15 mg of NaHCO₃, and (iv) 30 mg of NaHCO₃. The bottle was sealed with a screwed valve in order to prevent leakage of the carbon dioxide. At equilibrium, under room temperature (approximately 30°C or less), the dry ice inside of the bottle was transformed into either a homogeneous gas state or a gas-liquid coexistent state. The bottle was allowed to remain at room temperature for approximately 18-20 hours.

The valve was then unscrewed, and the collagen was released from the stainless-steel bottle, allowing the hydrogel to return to atmospheric pressure. The gelling ability and handling properties of each of the released gels was analyzed, and the visual appearance of each gel was observed and photographed.

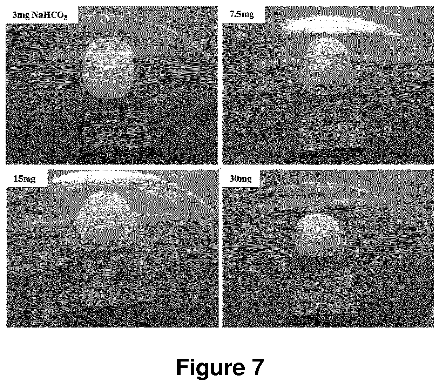

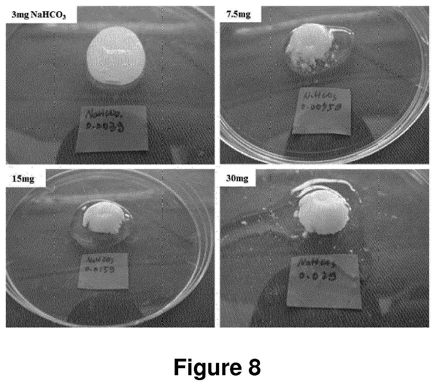

Table 5 (below) shows the results of gelling ability, handling property, and visual appearance of the gels released from the stainless-steel bottle containing collagen and solid carbon dioxide with the different concentrations of NaHCO₃. The appearances of the resulting gels are depicted in Figures 7 and 8. Figure 7 shows the appearance of collagen and solid carbon dioxide mixed with the different concentrations of NaHCO₃ after approximately 10 minutes of gelling in the syringe. Figure 8 depicts the appearance of collagen and solid carbon dioxide with the different concentrations of NaHCO₃ approximately 30 minutes after being pushed out of the syringe.

**Table 5. Gelling Ability and Handling of NaHCO₃ Gels**

| Example | 19 | 20 | 21 | 22 |
|---|---|---|---|---|
| Collagen(g) | 0.2 | 0.2 | 0.2 | 0.2 |
| CO2 (g) | 4 | 4 | 4 | 4 |
| Purified water(ml) | 10 | 10 | 10 | 10 |
| NaHCO3(mg) | 3 | 7.5 | 15 | 30 |
| Operation | No | No | No | No |
| OD450nm | 2.302 | 2.535 | 2.593 | 2.621 |
| Gelation property | 0 | + | + | + |

### Examples 23-26

A series of four hydrogels was formed by mixing 10 ml of water, 0.2 g of collagen, and 4 g of solid carbon dioxide with one of the following amounts of sodium hydroxide (NaOH) in a 20 ml stainless-steel bottle: (i) 0.4 mg of NaOH, (ii) 4 mg of NaOH, (iii) 20 mg of NaOH, and (iv) 40 mg of NaOH. The bottle was sealed with a screwed valve in order to prevent leakage of the carbon dioxide. At equilibrium, under room temperature (approximately 30°C), the dry ice inside of the bottle was transformed into either a homogeneous gas state or a gas-liquid coexistent state. The bottle was allowed to remain at room temperature for approximately 18-20 hours.

The valve was then unscrewed, and the collagen was released from the stainless-steel bottle, allowing the hydrogel to return to atmospheric pressure. The gelling ability and handling properties of each of the released gels was analyzed, and the visual appearance of each gel was observed and photographed.

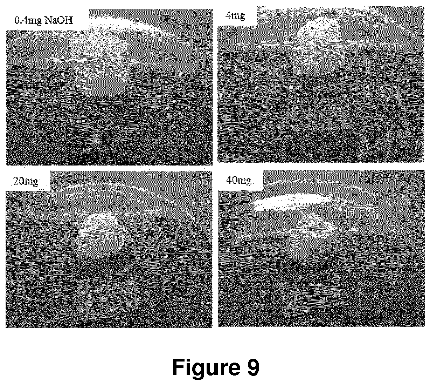

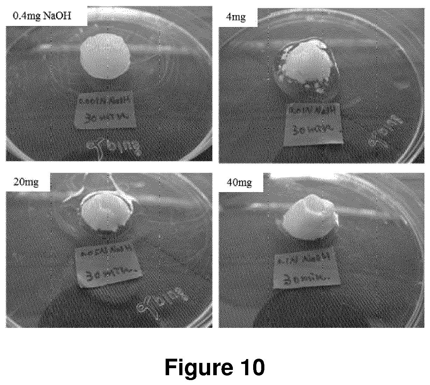

Table 6 (below) shows the results of gelling ability, handling property, and visual appearance of the gels released from the stainless-steel bottle containing collagen and solid carbon dioxide with the different concentrations of NaOH. The appearances of the resulting gels are depicted in Figures 9 and 10. Figure 9 shows the appearance of collagen and solid carbon dioxide mixed with the different concentrations of NaOH after approximately 10 minutes of gelling in the syringe. Figure 10 depicts the appearance of collagen and solid carbon dioxide with the different concentrations of NaOH approximately 30 minutes after being pushed out of the syringe.

**Table 6. Gelling Ability and Handling of NaOH Gels**

| Example | 23 | 24 | 25 | 26 |
|---|---|---|---|---|
| Collagen(g) | 0.2 | 0.2 | 0.2 | 0.2 |
| CO2 (g) | 4 | 4 | 4 | 4 |
| Purified water(ml) | 10 | 10 | 10 | 10 |
| NaOH(mg) | 0.4 | 4 | 20 | 40 |
| Operation | Yes | Yes | Yes | Yes |
| OD450nm | 2.4 | 2.587 | 2.657 | 2.701 |
| Gelation property | 0 | + | + | + |

### Example 27

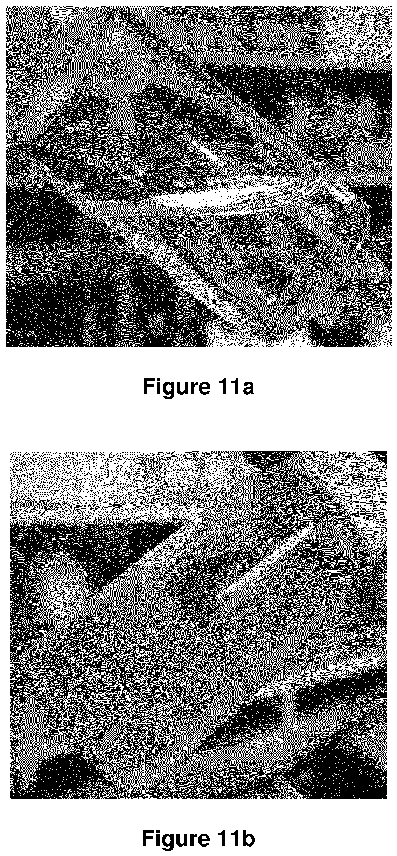

As discussed above, other polymers may be used instead of, or in addition to, collagen. For example, chitosan may be used in place of collagen. Chitosan powder may be dissolved at an acidic pH below 7 and may then begin precipitating out of solution to form a gel as the pH becomes less acidic and approaches neutral. In Example 27, 0.06 g of chitosan was dissolved into 3 ml of 0.1 M HCI to prepare a 2% chitosan solution, as shown in Figure 11a. The solution was incubated at a temperature of approximately 37°C to allow the chitosan to dissolve into the 0.1 M HCI. After 1 hour, 3 ml of 0.1 M NaHCO₃ was added to the chitosan solution to neutralize the solution. The mixture was then incubated at 37°C for two hours, and during that time, the chitosan precipitated out of solution and formed a chitosan gel, as is shown in Figure 11b.

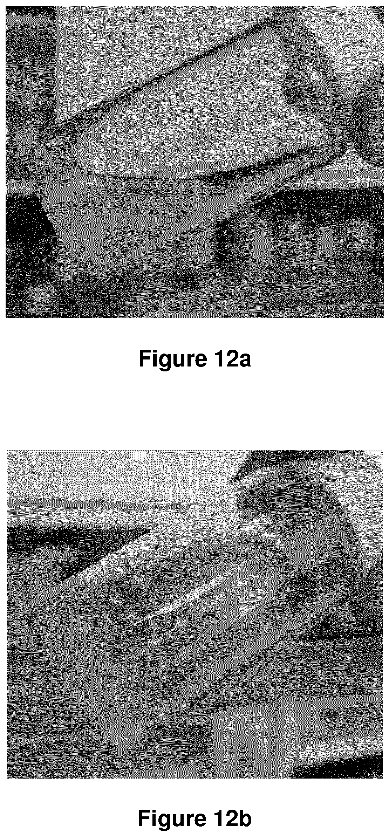

After the two-hour incubation period, the gel was mixed with 4 g of solid carbon dioxide in a 20 ml stainless steel bottle. The bottle was sealed with a screwed valve to inhibit leakage of carbon dioxide. The chitosan gel was incubated at room temperature for approximately 18 to 20 hours. During this time, the increased pressure caused the carbon dioxide to dissolve into solution, decreasing the pH value and causing the chitosan gel to dissolve back into solution. The chitosan hydrogel was then released from the stainless steel bottle, as shown in Figure 12a, and the visual appearance was observed. The chitosan hydrogel was incubated at 37°C following release. During that time, the chitosan solution transformed into a gel as the pH neutralized and the chitosan precipitated out of solution. Figure 12b depicts the visual appearance of the chitosan hydrogel in a gel after the two hours of incubation.

### Example 28

As discussed above, in some embodiments, the hydrogel may also serve as a delivery mechanism for a chemical compound. Such an exemplary hydrogel may deliver one or more pharmaceutical agents locally in the area to which the hydrogel is applied, e.g., to a target tissue region. This may allow a user to deliver a targeted dose of drug to a region of the body instead of, or in addition to, using the hydrogel to manipulate and/or repair tissue. In such embodiments, a pressurized hydrogel may be delivered to a region of the body, may be allowed to depressurize and gel in that body region, and may release a pharmaceutical agent contained in the hydrogel over a period of time as the hydrogel transitions from one state to another or as it degrades in the body. The pharmaceutical agent may be dispersed in the hydrogel when the hydrogel is in a depressurized gel state and may be released after the hydrogel is delivered to the target tissue region.

In one exemplary embodiment, caffeine was used as a model chemical compound to demonstrate the ability of hydrogels in accordance with this disclosure to act as a delivery mechanism for chemical compounds. In example 28, the hydrogel was formed by mixing 0.2 g of collagen, 4 g of solid carbon dioxide, and 10 ml of HBSS buffer with 0.0015 g of caffeine in a 20 ml stainless bottle. The bottle was sealed with a screwed valve in order to prevent leakage of the carbon dioxide. At equilibrium, under room temperature (approximately 30°C or less), the dry ice inside of the bottle was transformed into either a homogeneous gas state or a gas-liquid coexistent state. The bottle was allowed to remain at room temperature for approximately 18-20 hours.

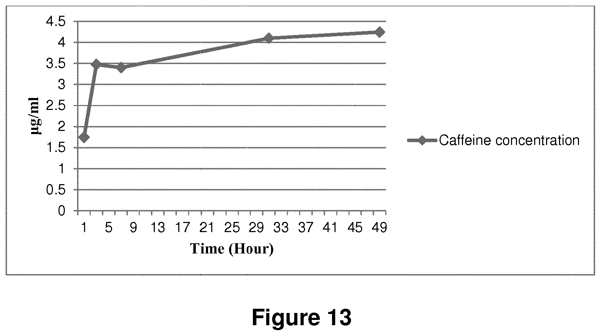

The valve was then unscrewed, the collagen was released from the stainless-steel bottle into a petri dish, and the resulting foamy material was allowed to cure at 37°C for 3 minutes. The cured hydrogel was then cut into 1 cm x 2 cm x 4 mm pieces. The pieces were then placed into a 50 ml tube together with 10 ml of ammonium acetate buffer and 20 µl of collagenase solution (10 mg/ml). The tube was then incubated in a water bath maintained at 37°C. Samples of supernatant were collected from the tube at a series of time points, and the concentration of caffeine in the supernatant sample at each time point was measured. The results of these measurements are shown in Figure 13.

Figure 13 demonstrates that the caffeine was released into the supernatant from the hydrogel over time. This time release of a chemical compound may mimic how the hydrogel would be degraded by collagenase inside of the human body. In this embodiment, approximately 80% of the caffeine in the hydrogel was released within the first 3 hours, while remaining caffeine was gradually released as the hydrogel degraded. The concentration of caffeine in the supernatant plateaued after about 24-48 hours of degradation in the collagenase solution, which may generally correspond with the time it takes for this specific hydrogel to degrade. This example demonstrates the ability of an exemplary hydrogel embodiment to deliver and release a drug in a predetermined amount. For example, in some embodiments, a hydrogel may deliver an initial burst release of a drug, e.g., due to the presence of drug absorbed on the surface of the set hydrogel, as well as a more gradual release of drug over time once applied to a target tissue region. It is believed that the drug is released as the hydrogel degrades, for example, by collagenase, within the body. While the caffeine example described above demonstrates the capacity for the disclosed hydrogel to act as a delivery mechanism, it is expected that the ability of the hydrogel to deliver a specific chemical compound may depend on, among other things, the properties of the specific hydrogel selected, the environment in which it is used, the nature and properties of the specific drug or combination of drugs, the dosing necessary for therapeutic benefits, and/or the intended therapeutic treatment, for example.

Embodiments of the present disclosure may provide several benefits over currently available hydrogels. For example, embodiments of the disclosure may provide a hydrogel capable of gelling in situ that is easy to manufacture and easy to transition between fluid and gel states using conventional processes. Many in-situ gelling hydrogels are controlled by temperature, which can require that the products be stored under regulated, low-temperature conditions prior to use. Moreover, the temperature may be difficult to control during use, and the hydrogel operation temperature window may be narrow. By using pressure to manipulate pH, embodiments of the present disclosure may offer hydrogel products that can be stored under room temperature conditions or under a wider range of temperatures. Further, in-situ gelling may be easily achieved once the pressurized condition is released and the hydrogel is re-exposed to ambient pressure.

The flowable and injectable nature of hydrogels according to the present disclosure make this type of hydrogel easy to transport, easy to deliver, and conformable for a wide variety of uses. These pressure-sensitive hydrogels are well-suited for applications that require the filling of three-dimensional areas. While other available hydrogels may be limited for use as a sealant and may require use of an accompanying scaffold, these pressure-sensitive hydrogels may be capable acting as both a sealant and a scaffold. Exemplary, three-dimensional uses may include medical applications, such as repairing wounds (e.g., after surgery or following resections, or after traumatic injuries such as gun shots) or filling defects, e.g., for bone or cartilage repair. In some embodiments, hydrogels may be used for dural repair or as dural substitutes, e.g., following excision of a brain tumor, craniotomy, or spinal surgery. They may also be used as three-dimensional scaffolds for biomedical and tissue engineering applications.

Further, because hydrogels of the present disclosure are porous, they may offer additional advantages over other hydrogel sealants. For example, many sealants adhere to the surrounding tissues, applying pressure to these tissues. The hydrogel may swell and expand in volume, causing pressure when in biological conditions. This pressure may build up and cause damage to the surrounding tissue or may cause unwanted pressure differentials on either side of the sealant. The pressure-sensitive hydrogels disclosed herein form tiny bubbles within the gel as it sets. The bubbles may not connect, and so while the gel may provide an adequate sealant, the porous nature of the hydrogel in the gel state may reduce or eliminate the pressure otherwise caused by conventional sealants. This may be particularly useful in medical applications, for example, when used in dural repair, so as to not apply pressure to portions of the central nervous system.

In addition to being biocompatible, hydrogels of the present disclosure also have tissue adhesion properties. Tissue may not be able to re-grow into most of the currently available hydrogels, so even when used with scaffolding, traditional hydrogels may stay separate within the body of a patient. Alternatively, tissue may be able to grow into embodiments of these novel pressure-sensitive hydrogels. This may allow for more natural, and/or stronger healing and regrowth over time, again providing advances in medical and three-dimensional applications.

Hydrogels of the present disclosure may also be used to manipulate, move, or stabilize tissue, to lift and/or separate tissue or layers of tissue (e.g., to aid in resection), or for tissue augmentation. Hydrogels may be used to coat, encapsulate, absorb, or dissolve substances, or to act as a barrier to moisture, for example. Hydrogels may also be used for treatment, for example, to aid in drug delivery, including, e.g., localized and/or slow-release delivery. Use for treatment may be alone or in combination with one of the above uses (e.g., to fill a wound and deliver an antiseptic).

In medical applications, a pressurized, fluid hydrogel may be delivered to an area of the body, and upon delivery, it may be exposed to a lower pressure, e.g., ambient pressure. Exposure to ambient pressure may cause the acid gas to come out of solution, causing the hydrogel to gel within the body. The acid gas that comes out of solution as the hydrogel sets may then be metabolized by the body, may be either passively or actively removed from the body, or may be left within the body. This may depend on the type of acid gas used. For example, if carbon dioxide is chosen as the acid gas, it may be metabolized by the body, because carbon dioxide is a common metabolite. In some embodiments, the resulting hydrogel may not be directly ejected into the body. Accordingly, after ejection, much of the acid gas may dissipate into atmosphere, with only a portion of the acid gas being trapped inside the hydrogel. Additionally, the remaining acid gas may be released over a period of time as the hydrogel degrades. In such embodiments, there may not be a burst of acid gas at the initial stage of hydrogel delivery and implantation, since most of the acid gas will dissipate into the atmosphere or will only slowly release over time. The smaller quantity of remaining acid gas may have no significant effect on the body when it is slowly released as the hydrogel degrades. In some embodiments, though, this remaining quantity may be actively removed from the body, or, in the case of gases such as carbon dioxide, may be metabolized by the body.

In some embodiments, other materials may be included in the hydrogel. For example, dyes may be added to aid in user visualization. Pharmaceutical agents may be included in the hydrogel to achieve localized and/or time-released delivery of treatment to the surrounding area as the hydrogel degrades within the body. Examples of such agents may include anesthetics, anti-inflammatories, antiseptics, or medications that facilitate tissue regeneration, prevent infection (e.g., antibiotics), or treat diseases (e.g., cancer). Other additive materials may include chemicals, ceramics, and biomaterials, such as growth factor, fibrinogen, platelet-rich plasma, or other suitable materials or combination of materials. In some embodiments, chemicals capable of controlling or adjusting pH and osmolality, such as, e.g., sodium bicarbonate, sodium chloride, calcium chloride, or potassium chloride, may be included. Such additives may act as a buffer, e.g., as the hydrogel degrades and the acid gas is released.

## Claims

1. A pressurized hydrogel composition, comprising:
a liquid solvent;
a polymer; and
an acid gas,
wherein the pressurized hydrogel composition is in a liquid state, and
wherein the pressurized hydrogel composition is capable of forming a gel state when depressurized.

2. The pressurized hydrogel composition of claim 1, wherein the liquid solvent includes at least one of water or buffer solution.

3. The pressurized hydrogel composition of claim 1, wherein the polymer includes collagen.

4. The pressurized hydrogel composition of claim 1, wherein the acid gas includes carbon dioxide.

5. A composition comprising:
a pressurized hydrogel composition, comprising:
a liquid solvent;
a polymer; and
an acid gas,
wherein the pressurized hydrogel composition is in a liquid state, and
wherein the pressurized hydrogel composition is capable of forming a gel state when depressurized.

6. The composition of claim 5, wherein the polymer includes collagen.

7. The composition of claim 5, wherein the acid gas includes carbon dioxide.

8. The composition of claim 5, further comprising a pharmaceutical agent.

9. The composition of claim 8, wherein when depressurized, the pharmaceutical agent is distributed throughout the gel.

10. The composition of claim 5, wherein the composition has a first pH when in the liquid state and has a second pH when in the gel state, and the first pH is lower than the second pH.

11. A method of making a pressure-sensitive hydrogel composition, comprising:
exposing a polymer and a solvent to an acid gas; and
subjecting the polymer, the solvent, and the acid gas to an increase in pressure to dissolve the acid gas in the solvent to form a more acidic solution,
wherein the more acidic solution causes the polymer to dissolve into the solution to form a fluid.

12. The method of claim 11, wherein the polymer includes collagen, and the acid gas includes carbon dioxide.

13. A pressurized hydrogel composition according to claim 1 for use in repairing tissue, comprising:
delivering the pressurized hydrogel composition to a target tissue region; and
depressurizing the pressurized hydrogel composition,
wherein the pressurized hydrogel composition is in the liquid state with the acid gas and the polymer dissolved in the solvent, and
wherein the depressurized hydrogel composition is in the gel state with the acid gas and the polymer substantially precipitated out of the solvent, wherein precipitation of the polymer out of the solvent causes the composition to transition to the gel state.

14. The composition of claim 13, wherein the pressurized hydrogel composition has a lower pH than the depressurized hydrogel composition.

15. The composition of claim 13, wherein depressurizing the pressurized hydrogel composition includes exposing the pressurized hydrogel composition to ambient conditions.

16. The composition of claim 13, wherein the pressurized hydrogel composition further includes a pharmaceutical agent, wherein the pharmaceutical agent is dispersed in the gel when the pressurized hydrogel composition is depressurized, and the pharmaceutical agent is released after the pressurized hydrogel composition is delivered to the target tissue region.

## Patentansprüche

1. Unter Druck stehende Hydrogelzusammensetzung, umfassend:
ein flüssiges Lösungsmittel;
ein Polymer; und
ein saures Gas,
wobei sich die unter Druck stehende Hydrogelzusammensetzung in einem flüssigen Zustand befindet, und
wobei die unter Druck stehende Hydrogelzusammensetzung dazu in der Lage ist, einen Gelzustand zu bilden, wenn druckentlastet.

2. Unter Druck stehende Hydrogelzusammensetzung nach Anspruch 1, wobei das flüssige Lösungsmittel zumindest eines von Wasser oder Pufferlösung beinhaltet.

3. Unter Druck stehende Hydrogelzusammensetzung nach Anspruch 1, wobei das Polymer Kollagen beinhaltet.

4. Unter Druck stehende Hydrogelzusammensetzung nach Anspruch 1, wobei das saure Gas Kohlendioxid beinhaltet.

5. Zusammensetzung, umfassend:
eine unter Druck stehende Hydrogelzusammensetzung, umfassend:
ein flüssiges Lösungsmittel;
ein Polymer; und
ein saures Gas,
wobei sich die unter Druck stehende Hydrogelzusammensetzung in einem flüssigen Zustand befindet, und
wobei die unter Druck stehende Hydrogelzusammensetzung dazu in der Lage ist, einen Gelzustand zu bilden, wenn druckentlastet.

6. Zusammensetzung nach Anspruch 5, wobei das Polymer Kollagen beinhaltet.

7. Zusammensetzung nach Anspruch 5, wobei das saure Gas Kohlendioxid beinhaltet.

8. Zusammensetzung nach Anspruch 5, ferner umfassend einen pharmazeutischen Wirkstoff.

9. Zusammensetzung nach Anspruch 8, wobei der pharmazeutische Wirkstoff, wenn druckentlastet, im gesamten Gel verteilt ist.

10. Zusammensetzung nach Anspruch 5, wobei die Zusammensetzung im flüssigen Zustand einen ersten pH-Wert aufweist und im Gelzustand einen zweiten pH-Wert aufweist, und der erste pH-Wert niedriger als der zweite pH-Wert ist.

11. Verfahren zur Herstellung einer druckempfindlichen Hydrogelzusammensetzung, umfassend:
Aussetzen eines Polymers und eines Lösungsmittels einem sauren Gas; und
Unterwerfen des Polymers, des Lösungsmittels und des sauren Gases einer Druckerhöhung, um das saure Gas in dem Lösungsmittel aufzulösen, um eine saurere Lösung zu bilden,
wobei die saurere Lösung bewirkt, dass sich das Polymer in der Lösung auflöst, um ein Fluid zu bilden.

12. Verfahren nach Anspruch 11, wobei das Polymer Kollagen beinhaltet und das saure Gas Kohlendioxid beinhaltet.

13. Unter Druck stehende Hydrogelzusammensetzung nach Anspruch 1 zur Verwendung bei der Gewebereparatur, umfassend:
Abgeben der unter Druck stehenden Hydrogelzusammensetzung an eine Zielgeweberegion; und
Druckentlasten der unter Druck stehenden Hydrogelzusammensetzung,
wobei sich die unter Druck stehende Hydrogelzusammensetzung im flüssigen Zustand befindet, wenn das saure Gas und das Polymer in dem Lösungsmittel aufgelöst sind, und
wobei sich die druckentlastete Hydrogelzusammensetzung im Gelzustand befindet, wenn das saure Gas und das Polymer im Wesentlichen aus dem Lösungsmittel ausgefällt sind, wobei Ausfällung des Polymers aus dem Lösungsmittel bewirkt, dass die Zusammensetzung in den Gelzustand übergeht.

14. Zusammensetzung nach Anspruch 13, wobei die unter Druck stehende Hydrogelzusammensetzung einen niedrigeren pH-Wert als die druckentlastete Hydrogelzusammensetzung aufweist.

15. Zusammensetzung nach Anspruch 13, wobei das Druckentlasten der unter Druck stehenden Hydrogelzusammensetzung das Aussetzen der unter Druck stehenden Hydrogelzusammensetzung Umgebungsbedingungen beinhaltet.

16. Zusammensetzung nach Anspruch 13, wobei die unter Druck stehende Hydrogelzusammensetzung ferner einen pharmazeutischen Wirkstoff beinhaltet, wobei der pharmazeutische Wirkstoff in dem Gel dispergiert ist, wenn die unter Druck stehende Hydrogelzusammensetzung druckentlastet wird, und der pharmazeutische Wirkstoff freigesetzt wird, nachdem die unter Druck stehende Hydrogelzusammensetzung an die Zielgeweberegion abgegeben wird.

## Revendications

1. Composition d'hydrogel sous pression, comprenant :
un solvant liquide ;
un polymère ; et
un gaz acide,
ladite composition d'hydrogel sous pression étant à l'état liquide, et ladite composition d'hydrogel sous pression étant capable de former un état de gel lorsqu'elle est dépressurisée.

2. Composition d'hydrogel sous pression selon la revendication 1, ledit solvant liquide comprenant au moins l'une parmi l'eau ou une solution tampon.

3. Composition d'hydrogel sous pression selon la revendication 1, ledit polymère comprenant du collagène.

4. Composition d'hydrogel sous pression selon la revendication 1, ledit gaz acide comprenant du dioxyde de carbone.

5. Composition comprenant :
une composition d'hydrogel sous pression, comprenant :
un solvant liquide ;
un polymère ; et
un gaz acide,
ladite composition d'hydrogel sous pression étant à l'état liquide, et
ladite composition d'hydrogel sous pression étant capable de former un état de gel lorsqu'elle est dépressurisée.

6. Composition selon la revendication 5, ledit polymère comprenant du collagène.

7. Composition selon la revendication 5, ledit gaz acide comprenant du dioxyde de carbone.

8. Composition selon la revendication 5, comprenant en outre un agent pharmaceutique.

9. Composition selon la revendication 8, lorsqu'elle est dépressurisée, ledit agent pharmaceutique étant réparti dans tout le gel.

10. Composition selon la revendication 5, ladite composition ayant un premier pH lorsqu'elle est à l'état liquide et ayant un second pH lorsqu'elle est à l'état de gel, et ledit premier pH étant inférieur au second pH.

11. Procédé de fabrication d'une composition d'hydrogel sensible à la pression, comprenant :
l'exposition d'un polymère et d'un solvant à un gaz acide ; et
la soumission du polymère, du solvant et du gaz acide à une augmentation de pression pour dissoudre le gaz acide dans le solvant pour former une solution plus acide,
ladite solution plus acide provoquant la dissolution du polymère dans la solution pour former un fluide.

12. Procédé selon la revendication 11, ledit polymère comprenant du collagène et ledit gaz acide comprenant du dioxyde de carbone.

13. Composition d'hydrogel sous pression selon la revendication 1 destinée à être utilisée dans la réparation d'un tissu, comprenant :
l'administration de la composition d'hydrogel sous pression à une région de tissu cible ; et
la dépressurisation de la composition d'hydrogel sous pression,
ladite composition d'hydrogel sous pression étant à l'état liquide avec le gaz acide et le polymère dissous dans le solvant, et
ladite composition d'hydrogel dépressurisée étant à l'état de gel avec le gaz acide et le polymère sensiblement précipité hors du solvant, la précipitation du polymère hors du solvant provoquant la transition de la composition à l'état de gel.

14. Composition selon la revendication 13, ladite composition d'hydrogel sous pression ayant un pH inférieur à celui de la composition d'hydrogel dépressurisée.

15. Composition selon la revendication 13, ladite dépressurisation de la composition d'hydrogel sous pression comprenant l'exposition de la composition d'hydrogel sous pression aux conditions ambiantes.

16. Composition selon la revendication 13, ladite composition d'hydrogel sous pression comprenant en outre un agent pharmaceutique, ledit agent pharmaceutique étant dispersé dans le gel lorsque la composition d'hydrogel sous pression est dépressurisée, et ledit agent pharmaceutique étant libéré après l'administration de la composition d'hydrogel sous pression à la région de tissu cible.
